# EUROPÄISCHE PATENTSCHRIFT

(11) **EP 2 859 917 B1**
(45) Veröffentlichungstag und Bekanntmachung des Hinweises auf die Patenterteilung: **08.08.2018**
(21) Anmeldenummer: 14154506.1
(22) Anmeldetag: 10.02.2014
(51) Int. Cl.: A61Q 3/02, A61K 8/92, A61K 8/25, A61K 8/34, A61K 8/37

(54) **Kosmetische Zusammensetzung und deren Herstellung**
Cosmetic composition and use of same
Composition cosmétique et sa fabrication

(43) Veröffentlichungstag der Anmeldung: 15.04.2015
(73) Patentinhaber: LOGOCOS Naturkosmetik AG, 31020 Salzhemmendorf (DE)
(72) Erfinder: Georgi, Sebastian, 31020 Salzhemmendorf (DE)
(74) Vertreter: Kröncke, Rolf

(56) Entgegenhaltungen:
- DE-A1- 10 050 814
- DE-U1- 20 018 602
- DE-U1- 20 109 224
- DE-U1-202009 000 749
- DE-U1-202013 009 976
- FR-A1- 2 964 568

## Beschreibung

Die vorliegende Erfindung betrifft kosmetische oder pharmazeutische Zusammensetzungen, insbesondere Nagellacke auf Schellackbasis. Diese Zusammensetzungen sind insbesondere farbige Nagellacke auf Schellackbasis weiterhin aufweisend Verdicker und wasserhaltiges Lösungsmittel sowie Alkohol. In einem weiteren Aspekt bezieht sich die vorliegende Erfindung auf Behälter enthaltend eine erfindungsgemäße kosmetische oder pharmazeutische Zusammensetzung, ausgestattet mit einer Applikationsvorrichtung zum Aufbringen der kosmetischen Zusammensetzung, insbesondere auf einen Nagel. Schließlich richtet sich die vorliegende Erfindung auf ein Verfahren zur Herstellung einer solchen Zusammensetzung, insbesondere eines Nagellacks.

### Stand der Technik

Nagellack stellt ein bekanntes Kosmetikprodukt zum Lackieren von Finger- und Fußnägeln dar. Nagellacke werden auch in pharmazeutischem Bereich zum Aufbringen von Wirkstoffen, z.B. anti-mikrobielle oder anti-mykotische Wirkstoffe, verwendet. Nagellack kann dabei als Klarlack oder als farbiger Lack eingesetzt werden. Auf dem Markt erhältliche Nagellacke haben Nitrozellulose als Basis für die filmbildende Grundlage. Nagellacke liegen üblicherweise in flüssiger Form vor, wobei nach Auftragen des Nagellacks auf die Nägel die im Nagellack vorliegenden Lösungsmittel verdampfen und auf dem Nagel ein Film ausgebildet wird. Mittelviskose Nitrocellulose ist in Estern als Lösungsmittel kolloidal löslich und bildet nach Verdampfen der Lösungsmittel einen transparenten und harten Film. Durch Zugabe von z.B. Toluolsulfonamid-Formaldehyd- oder Toluolsulfonamid-Epoxid-Harzen erfolgt Glanz und eine Festigung des Filmes. Zur Verstärkung von Glanz und Festigkeit werden häufig zusätzlich Polyacrylsäureester und/oder Polymethacrylsäureester eingesetzt. Der Verdunstungsprozess dauert einige Minuten bis der Lack ohne Zerstörung berührt werden kann. Weitere Zeit ist notwendig, bis die Kratzfestigkeit und Stoßfestigkeit des Lackfilms etabliert wird. Als Lösemittel von nitrocelluloserhaltigen Lacken werden z.B. Ethylacetat, Butylacetat, Methylethylketon oder dergleichen eingesetzt.

Als Gelbildner werden in herkömmlichen Nagellacken in der Regel Stearalkonium Hectorite oder Quaternium-18 Hectorite eingesetzt sowie anorganische Säuren, wie Propylencarbonat zur Ausbildung einer thixotropen Gelstruktur.

Zur Reduzierung der Härte werden Stoffe wir Campher, Acetyltributylcitrat oder Dibutylphtalat als Weichmacher zugesetzt. Zusatzstoffe, wie Farbstoffe umfassen Pigmente wie Titandioxid, Eisenoxid und andere organische Pigmente, wie Monoazofarbstoffe. Weitere Zusatzstoffe sind Verdicker, Antioxidantien und übliche weitere Zusatzstoffe. Ein Problem konventioneller Nagellacke ist die Vielzahl der Gefahrstoffe, die in diesen Kosmetika enthalten sind, insbesondere auch toxikologische bedenkliche Stoffe. Es wurde daher versucht einige der kritischen Stoffe in diesen Nagellacken zu entfernen, wie Formaldehyd, Toluol, Phtalate und Kolophonium.

Wasserlösliche Nagellacke weisen als Basis Acrylate und Polyacrylate, sowie Styrene/Acrylene Copolymere ggf. weiterhin mit Silikonen wie Disiloxane oder ethoxilierte Silikonverbindungen wie PEG-12 Dimethicone, Polyurethanen, Polyvinylalkohole Vinylacetate versetzt, auf. Diese Lacke haben aber eine schlechte Haltbarkeit und sind schwierig zu entfernen. Darüber hinaus gibt es permanent Nagellacke, die auf Acryllacke basieren und meist mit Licht, z.B. UV-Licht, gehärtet werden. Diese Lacke bedürfen aber einer entsprechenden Vorbereitung der Nägel. Außerdem werden meist mehrere Schichten aufgetragen umfassend Haftvermittler, den Lack selbst, sowie Versiegler, um die Oberfläche des Lackes zu verbessern. Abgesehen davon, dass diese mehrschichtigen Konstruktionen häufig den mechanischen Belastungen nicht Stand halten, sind diese schwierig zu entfernen.

Die herkömmlichen Nagellacke entsprechen insbesondere nicht den Wünschen nach Naturkosmetika. Naturkosmetik muss entsprechenden Anforderungen auch durch die Verbraucher genügen. So wird eine 100 %ige biologische Abbaubarkeit angestrebt bzw. eine 100 %ige Verträglichkeit.

Aus der EP 1372578 ist ein kosmetischer oder pharmazeutischer Nagellack bekannt, der aus pflanzlichen Rohstoffen bestehen soll. Dieser farblose Nagellack ist einer, der zu mindestens 50 % aus nur reinem Alkohol, Schellack und Benzoe-Öl besteht.

Dabei wird Alkohol als Lösungsmittel und Schellack als Filmbildner eingesetzt. Verschiedene Naturharze, wie Benzoeharz, Myrrhenharz und Wachse wie Carnaubawachs in Verbindungen mit pflanzlichen Ölen, wie Rizinusöl und ätherischen Ölen, wie Kampfer, werden hinzugefügt.

Allerdings weist Schellack den Nachteil auf, dass dieser nur schwer auftragbar ist und der z.B. im Stand der Technik beschriebene Nagellack nur als Klarlack erhältlich ist. Die Einarbeitung von farbigen Pigmenten ist bisher nicht möglich, da sich die Pigmente im flüssigen Lack separieren und ein Aufbringen schwierig ist.

Eine Aufgabe der vorliegenden Erfindung besteht darin, Zusammensetzungen, insbesondere Nagellacke bereitzustellen, die die obigen Probleme verringern. Insbesondere ein Nagellack, der toxikologisch unbedenkliche Inhaltsstoffe aufweist und die Bereitstellung von insbesondere farbigen kosmetischen oder pharmazeutischen Zusammensetzungen, insbesondere farbigen Nagellacken, erlaubt und auch die sonstigen Anforderungen an Nagellacke erfüllen, nämlich eine rasche Trocknungszeit, gute Haltbarkeit, einfaches Entfernen mit speziellen Entfernern sowie den naturkosmetischen Anforderungen genügen.

### Beschreibung der Erfindung

Diese Aufgabe wird durch die kosmetische oder pharmazeutische Zusammensetzung, insbesondere in Form eines Nagellackes nach Anspruch 1 gelöst. In den Unteransprüchen werden Ausführungsformen hierzu weiter genannt. Weiterhin werden die Verwendungen solcher Zusammensetzungen in ein Applikationssystem zum Aufbringen der Zusammensetzung auf einen Nagel beschrieben sowie ein entsprechendes Verfahren zum Lackieren eines Nagels umfassend das Aufbringen der erfindungsgemäßen Zusammensetzung auf einen Nagel.

Es wird erfindungsgemäß ein Behälter enthaltend eine erfindungsgemäße kosmetische oder pharmazeutische Zusammensetzung bereitgestellt, wobei diese Zusammensetzung insbesondere als kosmetische Zusammensetzung z.B. in Form eines Nagellackes vorliegt. Dieser Behälter weist zusätzlich eine Applikationsvorrichtung, insbesondere einen Pinsel-Applikator zum Aufbringen der Zusammensetzung auf, insbesondere zum Aufbringen dieser Zusammensetzung, z.B. in Form eines Nagellackes auf einen Nagel.

Schließlich wird erfindungsgemäß ein Verfahren zur Herstellung einer kosmetischen oder pharmazeutischen Zusammensetzung, insbesondere eines Nagellackes, bereitgestellt. Dieses Verfahren umfasst die Schritte des Dispergierens von Pigmenten (Farbstoffen) in einem Dispersionsmittel, gefolgt von einem Vermahlen dieser Dispersion, um Pigmente mit einer Partikelgröße von kleiner 50 µm, wie kleiner 25 µm, zu erhalten. Der Verdicker wird in die vorgelegte Schellacklösung eingebracht und in diese Mischung aus Verdicker und Schellacklösung wird dann die Dispersion mit den vermahlenen Pigmenten eingeführt.

Am Markt sind Produkte, die eine dünne, haltbare, gleichmäßige Farbschicht auf die Nägel aufbringen, gewünscht. Der Lack sollte sich gut auftragen und verteilen lassen, schnell trocknen, gut halten und eine glänzende, glatte Oberfläche besitzen. Weiterhin sollte eine möglichst einfache und reibungslose Entfernung des Lackes möglich sein.

Erfindungsgemäß werden kosmetische oder pharmazeutische Zusammensetzungen, insbesondere Nagellacke bereitgestellt, die eine entsprechend geeignete Konsistenz besitzen, um insbesondere als farbige Nagellacke genutzt zu werden. In diesen farbigen Nagellacken liegen die enthaltenen Pigmente (Farbstoffe) derart stabilisiert vor, dass das Aufbringen eines gleichmäßigen, farbigen Films auf den Nagel möglich ist. Weiterhin entspricht die erfindungsgemäße Zusammensetzung, wie eine kosmetische Zusammensetzung, insbesondere in Form eines farbigen Nagellackes den Richtlinien der Naturkosmetik, d.h. es werden im Wesentlichen naturverträgliche Inhaltsstoffe eingesetzt.

Die erfindungsgemäße kosmetische Zusammensetzung gemäß Anspruch 1 umfasst dabei
15 bis 40 Gew.-% Schellack,
0,1 bis 10 Gew.-% Verdicker,
1 bis 40 Gew.-% wasserhaltiges Lösungsmittel,
0 bis 15 Gew.-% Füllstoffe;
0 bis 25 Gew.-% Farbstoffe;
ad 100% Alkohol.

Dabei bildet der Schellack in Form einer Schellacklösung die Basis der kosmetischen oder pharmazeutischen Zusammensetzung. Üblicherweise handelt es sich bei dem Schellack um eine alkoholische Schellacklösung, z.B. eine ethanolische Schellacklösung soweit nicht anders angegeben. Die Schellacklösung besteht z.B. aus entwachsten Blätterschellack, der in einem ethanolischen Biomassedestillat, z.B. einem Öko-Weizendestillat aufgelöst wurde. Üblicherweise ist das zum Lösen des Schellacks verwendete Lösungsmittel das gleiche Lösungsmittel, wie zum Auffüllen der Lösung verwendet, d.h. in einer Ausführungsform ist das Schellack eine alkoholische Schellacklösung, wobei der Alkohol der gleiche Alkohol ist, wie der verwendet zum Auffüllen auf 100 %. In einer Ausführungsform ist dieses Ethanol.

Die Zusammensetzung, die üblicherweise in einer flüssigen Form vorliegt, wird mit einem Alkohol aufgefüllt. Dieser Alkohol kann ein Ethanol, insbesondere ein Bioethanol sein oder andere alkoholische, insbesondere ethanolische Destillate aus Biomasse. Neben der Eigenschaft als Lösungsmitteln dient der Alkohol auch zur mikrobiologischen Stabilisierung.

Bevorzugt handelt es sich bei den Komponenten der erfindungsgemäßen Zusammensetzungen um Naturprodukte. D.h., die Zusammensetzung entspricht den Richtlinien der Naturkosmetik.

Der eingesetzte Schellack dient als Filmbildner auf dem Nagel und ist in einer Konzentration von 15 bis 40 Gew.-% Schellack, z.B. in einer Konzentration von 20 bis 30 Gew.-%, wie 23 bis 27 Gew.-% in der Zusammensetzung enthalten.

Weiterhin weist erfindungsgemäß die kosmetische oder pharmazeutische Zusammensetzung einen Verdicker auf. Vorliegend werden die Ausdrücke "Verdicker" und "Verdickungsmittel" synonym verwendet.

Das Verdickungsmittel wird zur Einstellung der Viskosität der Zusammensetzung, insbesondere des Nagellackes, verwendet. Bei dem Verdicker handelt es sich um ein Silikat, insbesondere eine amorphe Kieselsäure. Weitere geeignete Verdickungsmittel sind Hectorit, Bentonit und Magnesiumsilikat. In einer Ausführungsform liegen diese Verdicker nanoskalik vor, d.h. die Partikelgröße dieser Verdicker ist im Durchschnitt im Nanometerbereich. Z.B. kann die Partikelgröße im Durchschnitt unter 100 Nanometer liegen, wie z.B. unter 50 Nanometer, z.B. unter 25 Nanometer. Der Verdicker wird dabei in einer Menge von 0,1 bis 7 Gew.-%, z.B. in einer Menge von 1 bis 2,5 Gew.-%, eingesetzt.

Als weiterer Bestandteil liegt in der erfindungsgemäßen Zusammensetzung, insbesondere dem erfindungsgemäßen Nagellack, ein wasserhaltiges Lösungsmittel vor. Dieses wasserhaltige Lösungsmittel ist z.B. ein Wasserethyllactat-Gemisch. Ethyllactat weist eine geringe Toxizität und günstige Umwelteigenschaften auf und eignet sich daher besonders zur Verwendung in Naturprodukten. In der erfindungsgemäßen Mischung kann das Verhältnis von Wasser zu Ethyllactat variieren. Das wasserhaltige Lösungsmittel liegt in einer Menge von 1 bis 40 Gew.-%, wie in einer Menge von 7 bis 30 Gew.-%, z.B. 10 bis 25 Gew.-% vor. Das Verhältnis von Wasser zu Ethyllactat kann dabei von 1:1 bis 5:1 sein.

Im Falle von farbigen Zusammensetzungen, insbesondere farbigen Nagellacken, liegen weiterhin Farbstoffe (auch als Pigmente bezeichnet) vor. Diese Farbstoffe können in einer Menge von 1 bis 25 Gew.-%, wie 5 bis 15 Gew.-% vorliegen. Bei diesen Pigmenten handelt es sich in einer Ausführungsform um vermahlene Pigmente, z.B. Pigmente mit einer Partikelgröße von im Durchschnitt kleiner 50 µm, wie kleiner 25 µm.

Die kosmetische Zusammensetzung kann weiterhin weitere Füllstoffe enthalten, z.B. Talkum, Mika, Cellulose, Silikate, Bentonit, Hectorit, Montmorillonit. Wenn Füllstoffe vorhanden sind, liegen diese z.B. in einem Bereich von 0,5 bis 15 Gew.-%, wie 2 bis 8 Gew.-% vor.

Gegebenenfalls kann die Zusammensetzung, z.B. in Form eines Nagellackes, insbesondere eines flüssigen farbigen Nagellackes, weiterhin Zusatzstoffe enthalten, z.B. in einer Menge von 0,2 bis 10 Gew.-%. Diese Zusatzstoffe umfassen dabei solche aus den Gruppen der pflanzlichen Extrakte, ätherischen Öle bzw. Duft- oder Riechstoffe, anti-bakterielle oder anti-mykotische Wirkstoffe, Lösungsvermittler, Antioxidantien, Polyole, Harze und Wachse.

Beim Vorliegen von Wirkstoffen, wie anti-bakteriellen oder anti-mykotischen Wirkstoffen wird somit eine pharmazeutische Zusammensetzung bereitgestellt, insbesondere ein pharmazeutischer Nagellack.

In einer Ausführungsform handelt es sich bei der erfindungsgemäßen Zusammensetzung, z.B. in Form eines Nagellackes, die eine kosmetische oder pharmazeutische Zusammensetzung sein kann, eine bestehend aus
15 bis 40 Gew.-% Schellack;
0,1 bis 10 Gew.-% Verdicker;
1 bis 40 Gew.-% wasserhaltiges Lösungsmittel;
optional 0,5 bis 15 Gew.-% Füllstoffe;
optional 1 bis 25 Gew.-% Farbstoffe;
optional 0,2 bis 10 Gew.-% Zusatzstoffe;
ad 100 % Alkohol.

In einer Ausführungsform ist es insbesondere eine Zusammensetzung, wobei Schellack in einer Menge von 20 bis 30 Gew.-% und/oder der Verdicker in Form von Silikaten in einer Menge von 1 bis 2,5 Gew.-% und/oder das Lösungsmittel in einer Menge von 5 bis 25 Gew.-% und/oder ggf. vorhandenes Füllstoff in einer Menge von 2 bis 8 Gew.-% und/oder ggf. vorhandene Farbstoffe in einer Menge von 5 bis 15 Gew.-% enthalten ist und/oder der Alkohol ein Ethanol oder ethanolisches Destillat ist.

Die erfindungsgemäß verwendbaren Farbstoffe sind insbesondere Farbstoffe (Pigmente) ausgewählt aus Eisenoxid, Glimmerpigmente und Carmin.

Die erfindungsgemäße Zusammensetzung, insbesondere der erfindungsgemäße Nagellack ist dabei einer der eine gelartige Viskosität z.B. in einem Bereich von 2 dPa*S bis 14 dPa*s aufweist.

Eine entsprechende Konsistenz, nämlich eine leicht gelige Konsistenz in dem genannten Viskositätsbereichen, wird z.B. dadurch erzielt, dass der Verdicker in die Zusammensetzung und hier insbesondere in die Schellacklösung mit Hilfe hoher Scherkräfte, z.B. mittels Ultraturax eingebracht wird.

In der erfindungsgemäßen Zusammensetzung können zur Verbesserung der filmbildenden Eigenschaften weiterhin Wachse vorliegen, z.B. Bienenwachs oder pflanzliche Wachse und Harze, z.B. Styrax, Benzoe- oder Myrrhenharz, Elemi, Epoxidharze, Polyesterharze, Paraffine, Copolymere. Weiterhin können pflegende Komponenten zugesetzt sein, wie natürliche Öle, wie Rizinusöl, Mandelöl, Schwarzkümmelöl usw.

Die ggf. vorliegenden Wirkstoffe können z.B. ätherische Öle mit antimykotischer oder anti-mikrobieller Wirkung sein, z.B. Thymianöl, Neemöl, Rosenöl usw.

Wie ausgeführt handelt es sich bei der erfindungsgemäßen Zusammensetzung um eine kosmetische oder pharmazeutische Zusammensetzung, wobei diese Zusammensetzung insbesondere ein Nagellack ist, nämlich ein kosmetischer Nagellack oder pharmazeutischer Nagellack. In einer Ausführungsform handelt es sich insbesondere um einen kosmetischen farbigen Nagellack enthaltend Farbstoffe.

Bei der erfindungsgemäßen Zusammensetzung handelt es sich in einer Ausführung um eine Zusammensetzung, die den naturkosmetischen Kriterien des BDIH (Bundesverband der deutschen Industrie- und Handelsunternehmen) und Natrue entspricht.

Die erfindungsgemäße Zusammensetzung insbesondere in Form eines Nagellackes hat den Vorteil, dass sie den Standards der Naturkosmetik entspricht.

Sie ist insbesondere gut verteilbar mit einer relativ kurzen Trocknungszeit und weist ein gutes Anhaften auf dem Nagel auf unter Ausbildung eines entsprechenden Films auf Schellackbasis. Insbesondere erlaubt die erfindungsgemäße Zusammensetzung die Bereitstellung eines farbigen Nagellackes auf Schellackbasis.

Die Erfindung richtet sich daher auch auf die Verwendung einer erfindungsgemäßen kosmetischen oder pharmazeutischen Zusammensetzung als Nagellack insbesondere der Verwendung hiervon in einem Applikationssystem zum Aufbringen der kosmetischen Zusammensetzung auf einen Nagel.

Bereitgestellt wird weiterhin ein Verfahren zum Lackieren eines Nagels, wobei die erfindungsgemäße Zusammensetzung in Form eines Nagellackes, entweder klar oder farbig, auf einen Nagel aufgebracht wird.

Ein weiterer Aspekt der vorliegenden Erfindung richtet sich auf einen Behälter, die die erfindungsgemäße Zusammensetzung insbesondere den erfindungsgemäßen Nagellack enthält. Dieser Behälter weist eine Applikationsvorrichtung auf, die abnehmbar ausgestaltet ist. Bei dieser Applikationsvorrichtung handelt es sich insbesondere um einen Pinsel-Applikator. Diese Applikationsvorrichtung z.B. in Form eines Pinsel-Applikators ist zum Aufbringen der erfindungsgemäßen Zusammensetzung insbesondere in Form eines Nagellacks auf einen Nagel ausgebildet. Bei dem Behälter und dem Applikationssystem, z.B. dem Pinsel-Applikator handelt es sich um handelsübliche Applikatoren, die eine Verteilung des Lackes optimal erreichen. Z.B. besitzt dieser Applikator eine flache, breit Form mit abgerundeten Haaren, es können sowohl natürliche als auch synthetische Fasern eingesetzt werden.

Gegebenenfalls weist dieser Behälter zusätzlich eine Metallkugel auf. Diese Metallkugel erlaubt beim Aufschütteln des Lackes eine bessere Durchmischung hiervon, um ein gleichmäßiges Auftragen zu ermöglichen.

Schließlich stellt die vorliegende Erfindung ein Verfahren zur Herstellung der erfindungsgemäßen Zusammensetzung, insbesondere des erfindungsgemäßen Nagellacks bereit. Dieses Verfahren umfasst die folgenden Schritte: Das Begehren der Pigmente der Farbstoffe in einem Dispersionsmittel. Bei diesem Dispersionsmittel handelt es sich um übliche Dispersionsmittel z.B. solche bestehend aus Wasser und Alkohol, als um wasserhaltige Dispersionsmittel mit Wasser und mindestens einer zweiten Komponente. Diese mindestens zweite Komponente kann ein Alkohol einschließlich Ethanol aber auch Glycerin, Esterehtyllactat oder Sorbit sein.

Diese Dispersion wird vermahlen z.B. in einer Korbmühle, Stiftmühle oder Perlmühle oder mit Hilfe eines Walzwerkes, wobei die Pigmente eine Partikelgröße von kleiner 50 µm, z.B. kleiner 25 µm aufweisen.

Der Verdicker bzw. das Verdickungsmittel wird in die Schellacklösung eingebracht. Dieses erfolgt mit hoher Scherkraft z.B. mittels Ultraturax, um eine gelartige Konsistenz, d.h. um eine Lösung mit gewünschter Viskosität zu erhalten. In diese Lösung aus Schellack und Verdicker wird dann die Dispersion mit den vermahlenen Pigmenten eingerührt. Die weiteren Zusatzstoffe und Füllstoffe werden ebenfalls zu dieser viskosen Lösung gegeben. Dem Fachmann ist klar, wann er die entsprechenden Stoffe und in welcher Art und Weise er diese zuführt.

Diese oben genannten Verfahrensschritte erlauben die Bereitstellung der erfindungsgemäßen Zusammensetzung, insbesondere des erfindungsgemäßen farbigen Nagellackes in gewünschter Viskosität, um die gute Verteilbarkeit und einen einfachen Auftrag zu gewährleisten.

Im Folgenden wird die Erfindung anhand von Beispielen näher erläutert. Diese dienen lediglich zum besseren Verständnis der Erfindung ohne eine Beschränkung der Erfindung auf diese Beispiele darzustellen.

### Beispiele:

1.

| | Anteil in Gew.-% | Inhaltsstoff |
|---|---|---|
| Filmbildner | 26,6 | Shellac |
| Lösungsmittel | 18,6 | Mischung aus Wasser und Ethyllactat |
| Verdicker | 2,0 | Silika |
| Füllstoffe | 5,7 | Talkum, Mika |
| Farbstoffe | 7,3 | Mischung aus Eisenoxiden, Carmine, Titandioxid |
| Alkohol | 39,8 | |

2.

| | Anteil in Gew.-% | Inhaltsstoff |
|---|---|---|
| Filmbildner | 24,0 | Shellac |
| Lösungsmittel | 20,8 | Mischung aus Wasser und Ethyllactat |
| Verdicker | 1,5 | Silika |
| Füllstoffe | 4,5 | Talkum, Mika |
| Farbstoffe | 7,3 | Mischung aus Eisenoxiden, Carmine, Titandioxid |
| Zusatzstoff: | | |
| Parfümierung | | Duftstoffe, ätherische Öle |
| Alkohol | 40,9 | Ethanol und/oder Öko-Weizendestillat |

3.

| | Anteil in Gew.-% | Inhaltsstoff |
|---|---|---|
| Filmbildner | 20,0 | Shellac |
| Lösungsmittel | 10,5 | Mischung aus Wasser und Ethyllactat |
| Verdicker | 2,4 | Silika |
| Füllstoffe | 4,5 | Cellulose |
| Farbstoffe | 9,0 | Mischung aus Eisenoxiden, Carmine, Titandioxid und Glimmerpigmente |
| Zusatzstoff: | | |
| Parfümierung | 1,2 | Duftstoffe, ätherische Öle |
| Alkohol | 52,4 | Ethanol und/oder Öko-Weizendestillat |

4.

| | Anteil in Gew.-% | Inhaltsstoff |
|---|---|---|
| Filmbildner | 29,4 | Shellac |
| Lösungsmittel | 12,8 | Mischung aus Wasser und Ethyllactat |
| Verdicker | 2,1 | Silica |
| Füllstoffe | 8,2 | Talkum, Mika |
| Farbstoffe | 5,1 | Mischung aus Eisenoxiden, Carmine, Titandioxid und Glimmerpigmente |
| Zusatzstoff: | | |
| Parfümierung | 1,2 | Duftstoffe, ätherische Öle |
| Zusatzstoff: | | |
| Polyol | 2,0 | Glycerin |
| Alkohol | 39,2 | Ethanol und/oder Öko-Weizendestillat |

5.

| | Anteil in Gew.-% | Inhaltsstoff |
|---|---|---|
| Filmbildner | 25,2 | Shellac |
| Lösungsmittel | 14,0 | Mischung aus Wasser und Ethyllactat |
| Verdicker | 1,6 | Silika |
| Füllstoffe | 10,0 | Magnesiumsilikate |
| Farbstoffe | 10,3 | Mischung aus Eisenoxiden, Carmine, Titandioxid und Glimmerpigmente |
| Zusatzstoff: | | |
| Extrakte | 1,5 | Pflanzenextrakte |
| Alkohol | 37,4 | Ethanol und/oder Öko-Weizendestillat |

## Patentansprüche

1. Kosmetische oder pharmazeutische Zusammensetzung umfassend
15 bis 40 Gew.-% Schellack,
0,1 bis 10 Gew.-% Verdicker,
1 bis 40 Gew.-% wasserhaltiges Lösungsmittel,
0 bis 15 Gew.-% Füllstoffe;
0 bis 25 Gew.-% Farbstoffe;
ad 100% Alkohol, wobei der Verdicker ein Silikat, insbesondere amorphe Kieselsäure ist, und bevorzugt der Verdicker nanoskalik vorliegt.

2. Kosmetische oder pharmazeutische Zusammensetzung nach Anspruch 1 weiterhin enthaltend 0,2 bis 10 Gew.-% Zusatzstoffe, insbesondere pflanzliche Extrakte, ätherische Öle, Polyole, Lösungsvermittler, Antioxidantien, pH-Regulatoren, antibakterielle oder antimykotische Wirkstoffe.

3. Kosmetische oder pharmazeutische Zusammensetzung nach einem der vorhergehenden Ansprüche bestehend aus
15 bis 40 Gew.-% Schellack;
0,1 bis 10 Gew.-% Verdicker;
1 bis 40 Gew.-% wasserhaltiges Lösungsmittel;
optional 0,5 bis 15 Gew.-% Füllstoffe;
1 bis 25 Gew.-% Farbstoffe;
optional 0,2 bis 10 Gew.-% Zusatzstoffe;
ad 100 % Alkohol.

4. Kosmetische oder pharmazeutische Zusammensetzung nach einem der vorhergehenden Ansprüche, wobei Schellack in einer Menge von 20 bis 30 Gew.-% enthalten ist und der Alkohol ein Ethanol oder ethanolisches Destillat ist.

5. Kosmetische oder pharmazeutische Zusammensetzung nach einem der vorhergehenden Ansprüche, wobei der Verdicker insbesondere in Form von Silikaten in einer Menge von 1 bis 2,5 Gew.-% und der Alkohol als Ethanol oder ethanolisches Destillat enthalten ist.

6. Kosmetische oder pharmazeutische Zusammensetzung nach einem der vorhergehenden Ansprüche, wobei das Lösungsmittel ein Wasser-Ethyllactat Gemisch ist.

7. Kosmetische oder pharmazeutische Zusammensetzung nach einem der vorhergehenden Ansprüche, wobei die Farbstoffe ausgewählt sind aus Eisen-oxid, Glimmerpigmente, Titanoxid und Carmin.

8. Kosmetische oder pharmazeutische Zusammensetzung nach einem der vorhergehenden Ansprüche, wobei dieser ein Nagellack ist.

9. Verwendung einer kosmetischen oder pharmazeutischen Zusammensetzung nach einem der vorhergehenden Ansprüche als Nagellack.

10. Verwendung nach Anspruch 9 in einem Applikationssystem zum Aufbringen der kosmetischen oder pharmazeutischen Zusammensetzung auf einen Nagel.

11. Verfahren zum Lackieren eines Nagels mit dem Schritt des Aufbringens einer kosmetischen oder pharmazeutischen Zusammensetzung nach einem der Ansprüche 1 bis 8 auf einen Nagel.

12. Behälter enthaltend eine kosmetische oder pharmazeutische Zusammensetzung nach einem der Ansprüche 1 bis 8 und mit einer Applikationsvorrichtung, insbesondere einem Pinsel-Applikator, zum Aufbringen der kosmetischen oder pharmazeutischen Zusammensetzung, insbesondere auf einen Nagel.

13. Behälter nach Anspruch 12 zusätzlich enthaltend eine Metallkugel im Behälter.

14. Verfahren zur Herstellung einer kosmetischen oder pharmazeutischen Zusammensetzung nach Anspruch 1, insbesondere eines Nagellacks, umfassend die Schritte des Dispergierens von Pigmenten in einem Dispersionsmittel und Vermahlen dieser Dispersion, um Pigmente mit einer Partikelgröße von kleiner 50µm, bevorzugt kleiner 25µm zu erhalten; Einbringen des Verdickungsmittels in eine Schellacklösung; Einbringen der Dispersion mit vermahlenen Pigmenten in die Verdicker enthaltene Schellacklösung.

## Claims

1. Cosmetic or pharmaceutical composition, comprising
15 to 40 wt% of shellac;
0.1 to 10 wt% of thickener;
1 to 40 wt% of aqueous solvent;
0 to 15 wt% of fillers;
0 to 25 wt% of dyes;
the remainder up to 100% being alcohol, wherein the thickener is a silicate, in particular amorphous silica, and the thickener is preferably in nanoscale form.

2. Cosmetic or pharmaceutical composition according to Claim 1, further comprising 0.2 to 10 wt% of additives, in particular plant-based extracts, essential oils, polyols, solubilizers, antioxidants, pH regulators, or antibacterial or antifungal active agents.

3. Cosmetic or pharmaceutical composition according to either of the preceding claims, consisting of
15 to 40 wt% of shellac;
0.1 to 10 wt% of thickener;
1 to 40 wt% of aqueous solvent;
optionally 0.5 to 15 wt% of fillers;
1 to 25 wt% of dyes;
optionally 0.2 to 10 wt% of additives;
the remainder up to 100% being alcohol.

4. Cosmetic or pharmaceutical composition according to one of the preceding claims, wherein shellac is present in an amount of 20 to 30 wt% and the alcohol is an ethanol or ethanolic distillate.

5. Cosmetic or pharmaceutical composition according to one of the preceding claims, wherein the thickener is present in particular in the form of silicates in an amount of 1 to 2.5 wt% and the alcohol is present as ethanol or ethanolic distillate.

6. Cosmetic or pharmaceutical composition according to one of the preceding claims, wherein the solvent is a water/ethyl lactate mixture.

7. Cosmetic or pharmaceutical composition according to one of the preceding claims, wherein the dyes are selected from iron oxide, mica pigments, titanium oxide and carmine.

8. Cosmetic or pharmaceutical composition according to one of the preceding claims, wherein this composition is a nail varnish.

9. Use of a cosmetic or pharmaceutical composition according to one of the preceding claims as nail varnish.

10. Use according to Claim 9 in an application system for applying the cosmetic or pharmaceutical composition to a nail.

11. Method for applying varnish to a nail, comprising the step of applying a cosmetic or pharmaceutical composition according to one of Claims 1 to 8 to a nail.

12. Container containing a cosmetic or pharmaceutical composition according to one of Claims 1 to 8 with an application device, in particular a brush applicator, for applying the cosmetic or pharmaceutical composition, in particular to a nail.

13. Container according to Claim 12, additionally containing a metal bead in the container.

14. Method for producing a cosmetic or pharmaceutical composition according to Claim 1, in particular a nail varnish, comprising the steps of dispersing pigments in a dispersant and grinding this dispersion in order to obtain pigments with a particle size of less than 50 µm, preferably less than 25 µm; introducing the thickener into a shellac solution; introducing the dispersion with ground pigments into the thickener containing shellac solution.

## Revendications

1. Composition cosmétique ou pharmaceutique comprenant :
15 à 40 % en poids de gomme laque,
0,1 à 10 % en poids d'épaississant,
1 à 40 % en poids de solvant contenant de l'eau,
0 à 15 % en poids de charges,
0 à 25 % en poids de colorants,
jusqu'à 100 % d'alcool, l'épaississant étant un silicate, notamment de la silice amorphe, et l'épaississant se présentant de préférence sous forme nanométrique.

2. Composition cosmétique ou pharmaceutique selon la revendication 1, contenant en outre 0,2 à 10 % en poids d'additifs, notamment d'extraits végétaux, d'huiles éthérées, de polyols, de solubilisants, d'antioxydants, de régulateurs du pH, d'agents actifs antibactériens ou antimycotiques.

3. Composition cosmétique ou pharmaceutique selon l'une quelconque des revendications précédentes, constituée par :
15 à 40 % en poids de gomme laque,
0,1 à 10 % en poids d'épaississant,
1 à 40 % en poids de solvant contenant de l'eau,
éventuellement 0,5 à 15 % en poids de charges,
1 à 25 % en poids de colorants,
éventuellement 0,2 à 10 % en poids d'additifs,
jusqu'à 100 % d'alcool.

4. Composition cosmétique ou pharmaceutique selon l'une quelconque des revendications précédentes, dans laquelle la gomme laque est contenue en une quantité de 20 à 30 % en poids, et l'alcool est de l'éthanol ou un distillat éthanolique.

5. Composition cosmétique ou pharmaceutique selon l'une quelconque des revendications précédentes, dans laquelle l'épaississant est notamment contenu sous la forme de silicates en une quantité de 1 à 2,5 % en poids et l'alcool sous la forme d'éthanol ou d'un distillat éthanolique.

6. Composition cosmétique ou pharmaceutique selon l'une quelconque des revendications précédentes, dans laquelle le solvant est un mélange eau-lactate d'éthyle.

7. Composition cosmétique ou pharmaceutique selon l'une quelconque des revendications précédentes, dans laquelle les colorants sont choisis parmi l'oxyde de fer, les pigments à base de mica, l'oxyde de titane et le carmin.

8. Composition cosmétique ou pharmaceutique selon l'une quelconque des revendications précédentes, dans laquelle celle-ci est un vernis à ongles.

9. Utilisation d'une composition cosmétique ou pharmaceutique selon l'une quelconque des revendications précédentes en tant que vernis à ongles.

10. Utilisation selon la revendication 9 dans un système d'application pour l'application de la composition cosmétique ou pharmaceutique sur un ongle.

11. Procédé de vernissage d'un ongle comprenant l'étape d'application d'une composition cosmétique ou pharmaceutique selon l'une quelconque des revendications 1 à 8 sur un ongle.

12. Contenant contenant une composition cosmétique ou pharmaceutique selon l'une quelconque des revendications 1 à 8 et un dispositif d'application, notamment un applicateur à pinceau, pour l'application de la composition cosmétique ou pharmaceutique, notamment sur un ongle.

13. Contenant selon la revendication 12, contenant en outre une bille métallique dans le contenant.

14. Procédé de fabrication d'une composition cosmétique ou pharmaceutique selon la revendication 1, notamment d'un vernis à ongles, comprenant les étapes suivantes : la dispersion de pigments dans un dispersant et le broyage de cette dispersion afin d'obtenir des pigments d'une taille de particules inférieure à 50 µm, de préférence inférieure à 25 µm ; l'introduction de l'épaississant dans une solution de gomme laque ; l'introduction de la dispersion contenant les pigments broyés dans la solution de gomme laque contenant l'épaississant.
